# EUROPEAN PATENT SPECIFICATION

(11) **EP 4 197 521 B1**
(45) Date of publication and mention of the grant of the patent: **04.02.2026**
(21) Application number: 22211753.3
(22) Date of filing: 06.12.2022
(51) Int. Cl.: A61K 8/49, A61K 8/9789, A61Q 19/00, A61Q 19/08

(54) **COMPOSITION WITH SKIN ANTI-AGING ACTIVITY**
ZUSAMMENSETZUNG MIT HAUTALTERUNGSHEMMENDER WIRKUNG
COMPOSITION AYANT UNE ACTIVITÉ ANTI-VIEILLISSEMENT DE LA PEAU

(30) Priority: 14.12.2021 IT 202100031346
(43) Date of publication of application: 21.06.2023
(73) Proprietor: Giuliani S.p.A., 20129 Milano (IT)
(72) Inventor: GIULIANI, Giammaria, 6926 Montagnola (CH); RINALDI, Fabio, 20122 Milano (IT); PINTO, Daniela, 20151 Milano (IT); MARZANI, Barbara, 27020 Carbonara al Ticino (IT)
(74) Representative: Cattaneo, Elisabetta

(56) References cited:
- WO-A1-2006/101119
- JP-A- 2009 256 269
- KR-A- 20030 086 776
- US-A1- 2013 266 676
- PANDITA NARAHARI RAJANIGHANTAUH: "Haritakk Guna", KEY ATTRIBUTES OF TKDL, 12 October 1998 (1998-10-12), pages 1 - 3, XP093135652, Retrieved from the Internet <URL:file:///C:/Users/AS21418/Downloads/2023-07-06_TDOCNPL_EP22211753.pdf> [retrieved on 20240228]

## Description

### FIELD OF THE INVENTION

The present invention relates to a composition with a skin anti-aging activity.

The present invention originates in the cosmetic and food supplements sectors, in particular in the sector of products with anti-aging action.

Specifically, the present invention relates to a composition that acts on some biological mechanisms of the human body that intervene in the physiological skin aging process, whose activation results in an improvement of skin trophism and an improvement in its external appearance.

### STATE OF THE ART

Aging of the human body is a progressive natural degenerative process caused mainly by a combination of factors of genetic origin, environmental factors from the individual lifestyle.

The combination of these factors influences the speed of the aging process which affects all tissues of the human body and is accompanied by some symptoms and signs.

In this context, also skin aging is a dynamic physiological process with alterations mainly affecting the external tissues of the human body that begin to manifest themselves starting from the third decade of the individual's life.

Skin aging is mainly the result of the combination of a gradual decline in its functions and the accumulation of damage caused by environmental factors on the skin cellular and extracellular structures.

The typical signs of skin aging are mainly caused by a deterioration of the skin components, loss of cell turnover and alterations in the fibrous components.

On a biological level, one of the main causes of skin aging is to be found in a reduction in the formation of collagen, a protein that represents about 30% by weight of the body proteins and which has a structuring and filling action.

This reduction in collagen is mainly due to the decrease in the number of fibroblasts and the functional and metabolic capacity thereof. At the same time, there is also a reduction in the number of mast cells and in their ability to repair tissue damage.

These phenomena contribute to a thickening of the extracellular matrix and collagen fibers.

The action of external factors, mainly UV radiation, smog, exposure to particulate matter and high or low temperatures, is added to the action of these endogenous factors.

Among external agents, exposure to ultraviolet rays is one of the elements that most contribute to accelerating skin aging. For this reason, photoaging remains one of the main targets of anti-aging cosmetics.

It has been observed how exposure to UV radiation results in the formation of reactive oxygen species (ROS) and alterations to cellular homeostasis. These effects in turn alter the signal transduction pathways and the inflammatory cascade, thus inducing remodeling of the extracellular matrix.

These effects, together with the thinning and loss of elasticity of the skin and skin appendages, further contribute to accelerate the appearance of skin aging signs such as wrinkles, atrophy, hypotonicity, skin depressions, roughness, elastosis. There are currently numerous cosmetic products that aim to counteract and reduce these signs of skin aging. WO2006/101119 discloses a collagen improver extract from fruits of myrobalan with active ingredients being chebulagic and chebullic acid.

Skin moisturizing products are one of the most widespread categories of anti-aging cosmetics.

These products contain moisturizing and emollient substances capable of causing a temporary skin softening and a slight skin relaxation which makes facial roughness less apparent.

Products with moisturizing action are flanked by cosmetic products containing biologically active substances such as vitamins, for example vitamins A, C and **E,** with an antioxidant action that help to slow down skin damage resulting from photoexposure.

Another category of cosmetic products contains moisturizing substances combined with substances having a filling action, such as hyaluronic acid and collagen. However, these products do not provide satisfactory results as they are unable to penetrate the deep layers of the skin and generate the filling effect typical of fillers. Finally, fillers are substances that perform a double filling and relaxing action that considerably reduce facial wrinkles. However, fillers are mainly intended for aesthetic medicine and therefore do not fall under cosmetic products.

Currently, the need is therefore felt to have new cosmetics that are provided with an effective anti-aging action when applied to skin affected by the main signs of skin aging.

One of the aims of the invention is therefore to provide a composition that is effective in preventing and reducing the signs of skin aging, such as wrinkles or loss of skin elasticity.

Another aim of the invention is to provide a composition for the prevention and treatment of signs of skin aging containing one or more active ingredients that can be used both for topical application and for oral administration, and which are practically free of side effects.

### SUMMARY OF THE INVENTION

The Applicant, within the context of some research activities on compounds and plant extracts that target vascular endothelial growth factor A (VEGFA), found that extracts from the *Terminalia Chebula* plant, and some specific tannins contained therein, exert an inhibitory action on the so-called mTOR, a pathway that intervenes in some body biological processes involved in the cellular aging process.

Starting from this observation, the inventors unexpectedly found that specific tannins, particularly those extracted from a plant species, exert a slowing action on the natural skin aging process.

The invention originates from this observation and from the possibility of formulating the specific tannins identified in forms for oral or topical administration.

According to a first aspect, the present invention provides the cosmetic use of a composition comprising a tannin and a physiologically acceptable carrier for reducing or delaying the signs of skin aging or an aesthetic skin damage, wherein the tannin comprises chebulagic acid and said composition is a topical composition and comprises plantaricin, Vaccinum macrocarpon extract and hyaluronic acid or a physiologically acceptable salt thereof.

According to a preferred embodiment, chebulagic acid is of vegetable origin and may be obtained by extraction from *Terminalia Chebula* plant.

According to other embodiments of the invention, the tannin comprises chebulinic acid, optionally with chebulagic acid.

Advantageously, the cosmetic composition described herein is a skin anti-aging composition.

Within the scope of applications in the field of the present invention, the composition of the invention finds specific use in preventing or treating signs of skin aging, in particular the signs caused by exposure to ultraviolet radiation which contributes to cause premature skin aging.

According to some embodiments, the composition described herein is a topical composition for application to the skin, in particular on the face. According to this aspect, the composition is not for therapeutic use and is for cosmetic use in preventing and/or treating senescent skin, skin aging including signs of chronological aging and those caused by exposure to external agents, such as ultraviolet radiation.

According to another aspect, the composition is an anti-aging composition for oral administration containing at least one tannin as described above, suitable for slowing down and reducing the signs of skin aging in the human body.

### BRIEF DESCRIPTION OF THE DRAWINGS

The features and advantages of the present invention will be more apparent from the accompanying drawings, in which:
Figure 1 illustrates a schematic representation of chebulagic acid regulating activity on the promotion of autophagy mediated by an inhibitory action on mTOR;
Figures 2 A and B show bar graphs illustrating the relative quantification of VEGF-A (vascular endothelial growth factor A) respectively after 24h A) and 48h B) of incubation with chebulagic acid in the NCTC2544 human keratinocyte model;
Figures 3 A and B show bar graphs illustrating the relative quantification of mTOR after 24h A) and 48h B) of incubation with chebulagic acid in the NCTC2544 human keratinocyte model.

### DETAILED DESCRIPTION OF THE INVENTION

According to some aspects, the present invention originates from having found that chebulagic acid, and an extract from *Terminalia Chebula* plant containing chebulagic acid, have an anti-aging activity as claimed in the enclosed set of claims. Surprisingly, this anti-aging activity manifests itself clearly on the skin, resulting in a rapid and significant improvement of the main blemish typical of skin aging.

In one aspect, the object of the present invention is the skin anti-aging use of a composition containing a tannin, comprising chebulagic acid, advantageously obtainable by extraction from *Terminalia Chebula* plant , being said composition a topical composition and comprising plantaricin, Vaccinum macrocarpon extract and hyaluronic acid or a physiologically acceptable salt thereof.

The tannin is chebulagic acid, a natural analogue of rapamycin, a substance with anti-aging activity that acts through mTORC1 inhibition. The latter is a serine/threonine specific protein kinase, belonging to the family of kinases related to phosphatidylinositol-3 kinase (PI3K), an enzyme that plays an important role in the human body aging process.

The main functions of the mTOR signaling pathway are as follows: a) cell growth and proliferation; b) angiogenesis, (VEGF); c) bioenergetic action with availability and amplification of metabolism nutrients, and cell survival with repair and decrease of autophagy and apoptosis.

The mTOR signaling pathway mainly uses growth factors as the initial signaling molecule required for activation. These growth factors bind to tyrosine kinase receptors in the cell membrane, thus activating the pathway.

Many of the enzymes involved in this pathway are activated by phosphorylation. When growth factors bind to receptors on the membrane, a cascade of reactions is triggered that leads mTOR to activate many different transcription factors, which reach the cell nucleus and result in the transcription and expression of genes involved in cell growth and metabolism.

Typically, mTOR is the Target of Rapamycin, an antifungal macrolide molecule produced by a bacterium called *Streptomyces hygroscopicus,* isolated in 1970 on Easter Island.

mTOR functions as a component of a protein complex and there are two mTOR complexes: mTORC1 and mTORC2. These latter contain slightly different proteins, but all capable of stabilizing mTOR and regulating its activity, through inhibitory or activating regulatory mechanisms.

mTORC1 activates the ribosomal protein S6K, which induces protein synthesis and at the same time has an inhibitory action on 4EBP1, which normally inhibits protein synthesis.

It also increases lipid synthesis, mitochondrial biogenesis and inhibits autophagy.

Since mTOR has a significant role in the activation of genes related to cell growth, all failures in mTOR function may lead to abnormalities in cell replication; for example, many genes activated by mTORC1 and mTORC2 can prevent cell apoptosis and increase nutrient absorption, thus causing uncontrolled cell growth and tumor formation.

mTOR is therefore an extremely important cellular signaling pathway, as it is able to establish a communication link between the outside and the inside of the cell, through the regulation of transcription factors. (17), (18) (19), (20).

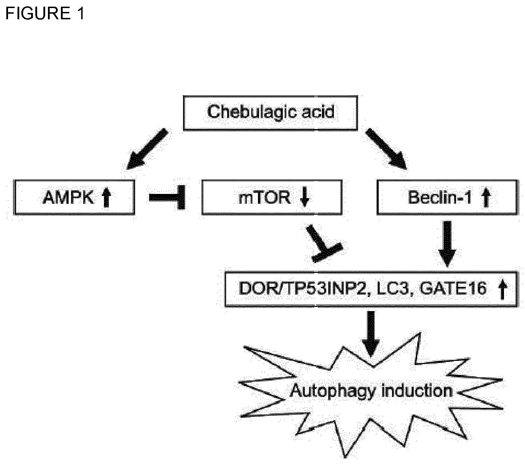

The mechanism of action of chebulagic acid, and of *Terminalia Chebula* extract titrated in chebulagic acid, is shown in Figure 1.

The biological activity of chebulagic acid, and of *Terminalia Chebula* extract containing it, are the basis for the reduction and prevention effects on the main aesthetic signs of skin aging, in particular those found on the face or neck of an individual. This effect is also achieved with chebulinic acid.

According to a first aspect of the invention, the cosmetic use of a composition as defined in the attached claim 1 is therefore provided.

A *Terminalia Chebula* extract or the biologically active compounds chebulagic acid, chebulinic acid or mixtures thereof, contained in this plant, preferably in leaves and fruits, may be used in the composition of the invention.

Embodiments of the non-therapeutic uses of the composition of the invention are defined in the attached dependent claims 2-8.

*Terminalia Chebula* Retz. (*T. chebula*), is a plant belonging to the *Combretaceae* family, a native plant of India and Southeast Asian countries. *Terminalia Chebula* extract contains tannins which are secondary phenolic metabolites. Typically, tannins have a relatively high molecular mass, for example from 500 to 20,000 Da, and have the capacity to form insoluble complexes with proteins, carbohydrates, nucleic acids and alkaloids.

In the formulation of the composition of the invention it is possible to use a *Terminalia Chebula* extract, advantageously titrated in tannins such as chebulagic acid and/or chebulinic acid.

The tannins, in particular chebulagic acid or chebulinic acid or mixtures thereof, used within the scope of the invention are preferably of vegetable origin, however they can also be of synthetic origin, or obtained by a chemical synthesis process. The plant extract of the invention may be obtained by extraction from a plant part or portion using a physiologically acceptable or edible solvent as an extraction medium.

The plant or the portion thereof may be used as a whole, ground, or preferably as an extract. Parts of the plant include fruit, leaves, inflorescences, roots, stem or trunk. Within the scope of the invention, the use of an extract from plant fruits or leaves is preferred.

The main biologically active compounds isolated and contained in a plant extract of *Terminalia Chebula* are the tannins chebulagic acid, chebulinic acid and mixtures thereof.

A further object of the present invention is the cosmetic use as defined in claim 9.

It is possible to obtain a plant extract from *Terminalia Chebula* by resorting to conventional extraction techniques, for example by maceration or solid-liquid techniques suitable for separating/extracting one or more biologically active components from vegetal tissues of plants and fruits thereof.

A suitable solvent for obtaining the plant extract of *Terminalia Chebula* is a physiologically acceptable liquid in which the biologically active components, typically tannins, are soluble.

Suitable solvents are polar solvents, preferably polar protic solvents, preferably water, a linear or branched C₁-C₆ alcohol, methanoic acid, formic acid, phenol, trifluoroethanoic acid, methanamide, diethylamine, and mixtures thereof.

Among these, suitable C₁-C₆ alcohols include methanol, ethanol, n-propanol, n-butanol, isopropanol, and mixtures thereof, optionally mixed with water to form hydroalcoholic solutions.

Preferred polar protic solvents are water, methanol, ethanol, and mixtures thereof. A preferred physiologically acceptable polar protic solvent is a hydroalcoholic mixture, preferably water-ethanol.

By way of example, the hydroalcoholic solvent may contain from 10% to 70% by volume of alcohol, preferably ethanol, preferably from 20% to 60% by volume, still preferably from 30% to 50% by volume, for example 40% by volume.

The extraction may be performed as cold or hot extraction, for example at 40-70 degrees centigrade.

The extraction of the biologically active components present in the plant vegetal tissues in the solvent can thus take place by diffusion and osmosis.

In certain embodiments, the extraction is carried out using a weight ratio between solvent and plant matrix comprised between 1:10 and 10:1.

A suitable *Terminalia Chebula* extract may be fluid, soft or dry.

Aqueous, alcoholic or hydroalcoholic extraction is particularly suitable when using plant fruits as the starting material for the extraction.

For example, in the fluid extract, 1 mL of extract contains the biologically active components soluble in 1 g of vegetable drug, in the soft extract the solvent is partially evaporated, specifically until the extract wets a filter paper, in the dry extract the solvent is evaporated almost completely to obtain a powder.

In the formulation of the composition, a dry extract, advantageously from the plant fruits or leaves, is preferably used.

For example, an initial step in the preparation of a suitable dry extract provides that the plant or portions thereof, preferably fruits or leaves, are cleaned, dried and optionally cut into pieces or chopped, ground or pulverized. At a later stage, this material is extracted using conventional methods.

A suitable extraction provides that a portion or vegetable matrix, preferably fruits or leaves, are ground to give a powder from which the extraction is carried out using a suitable solvent as previously described, for example water or a water-ethanol mixture, or ethanol, for example from 40 to 80% ethanol, for a time suitable for enriching the solvent with one or more biologically active components, for example 10 minutes.

In certain embodiments, the maceration time may vary between 10 minutes and 48 hours, 1 and 5 hours.

In these conditions, the solvent extraction of the biologically active components present in the plant tissues occurs by diffusion and osmosis.

In certain embodiments, the extract obtained may be sonicated and centrifuged, for example at 3000 rpm for 4-6 minutes. The extract obtained is collected and may be diluted with an alcohol, typically ethanol, for example to obtain a final volume of 10 mL, if starting from 1 gram of initial powder.

The extracted liquid may be filtered, if necessary. At a later stage, the liquid extract is dried to obtain the biologically active component in the form of a dry powder. Drying can be achieved with conventional techniques such as air drying, by heating or spray-drying.

According to one embodiment, the extraction is carried out on *Terminalia Chebula* fruits, preferably dried. In an initial step, the fruits are chopped or ground, and the ground material (powder) obtained is wetted or soaked, preferably in distilled water, conveniently at a temperature of 20-25°C. Subsequently, the wet ground material is filtered, and the filtered material is lyophilized and collected in suitable containers. The presence of tannins in the collected material can be determined, for example, using the protocol of Mukherjee, P. K. (2002). Quality control of herbal drugs: An Approach to evaluation of botanicals. New Delhi: Business Horizons.

Additional methods for obtaining the *Terminalia Chebula* plant extract include extraction techniques by digestion, infusion, squeezing, decoction, percolation, counter-current extraction, Soxhlet extraction, extraction with supercritical gases such as CO₂, or ultrasound extraction.

According to some embodiments, a *Terminalia Chebula* dry extract contains an amount of chebulagic acid higher than 10% by weight, and this represents the main biologically active ingredient contained in the plant.

In certain embodiments, the *Terminalia Chebula* plant extract is present in the composition in an amount from 0.1 to 90%, from 0.5 to 60%, from 1 to 30%, for example 10% by weight with respect to the total weight of the composition.

In certain embodiments, the composition of the invention contains a *Terminalia Chebula* plant extract in an amount ranging from 50 to 2000 mg, from 100 to 1200, from 200 to 600mg, for example 400 mg per dosage unit.

By way of example, a *Terminalia Chebula* extract with a water-based solution can contain from 5 to 25%, from 10 to 20% by weight of chebulagic acid.

In certain embodiments, chebulagic acid or chebulinic acid is present in the composition in an amount from 0.1 to 90%, from 0.5 to 60%, from 1 to 30%, for example 10% by weight with respect to the total weight of the composition.

The composition of the invention may be authorized on the market as a cosmetic product for local application, or as a dietary or nutritional supplement intended for oral administration.

Typically, the composition of the invention comprises a physiologically and/or cosmetically acceptable carrier, diluent, or excipient.

Typically, the physiologically or cosmetically acceptable carrier of the composition of the invention is an excipient, carrier, or diluent suitable for topical application and/or for oral administration.

Within the scope of the present invention, the term "carrier" refers to an excipient, carrier, diluent, or adjuvant that may be present in the composition of the invention. Any carrier and/or excipient suitable for the form of preparation desired for administration is contemplated in the uses of the plant extract or active ingredients present therein and described herein.

In certain embodiments, the composition of the invention contains one or more components of plant origin which are biologically active and substantially free of side effects, when administered orally or locally.

The physiologically or pharmaceutically carrier, diluent or excipient may be selected based on the route of administration for which the resulting pharmaceutical composition is intended.

According to some preferred embodiments, the composition may be in a form for oral administration.

The compositions for oral administration may be in solid or liquid form. Typical solid form compositions comprise tablets, capsules, powders, granulates, pills. Typical compositions in liquid form comprise solutions, emulsions, suspensions, syrups. All compositions also comprise controlled release forms thereof.

A preferred embodiment of the composition is the one in tablets or capsules.

The capsules or tablets generally comprise a suitable carrier or excipient in which the plant extract is dispersed, typically in dry form.

In this case, suitable excipients contained in the formulation are cellulose and derivatives thereof, such as hydroxymethylcellulose, hydroxypropylmethylcellulose, methylcellulose, hydroxypropylcellulose, hydroxyethylcellulose, carboxyethylcellulose, ethylhydroxyethylcellulose, carboxymethylcellulose, cellulose acetate butyrate, cellulose acetate phthalate, and mixtures thereof.

Further examples of suitable excipients comprise polymers belonging to the lactam family such as pyrrolidone and derivatives thereof, for example polyvinylpyrrolidone, polyvinylpolypyrrolidone and mixtures thereof, inorganic salts such as calcium or dicalcium phosphate, lubricants such as magnesium stearate, triacylglycerols and mixtures thereof.

The composition may further comprise one or more of the following excipients: bulking agents such as calcium phosphates, stabilizers such as carboxymethylcellulose, powdered hyaluronic acid, gums such as Tara gum, carbomer such as carbopol^{®}, glycerin, coating agents such as polyvinyl alcohol, polyethylene glycol, talc, anti-caking agents such as fatty acids magnesium salts; silicon dioxide.

The composition may also contain preservatives, for example sodium benzoate, potassium sorbate.

The *Terminalia Chebula* extract contained in the composition of the invention may be present in variable amount, for example ranging from 0.0001% by weight to 50% by weight, from 0.1% by weight to 20% by weight, typically from 0.5 to 5% by weight. According to certain embodiments, the composition of the invention further comprises one or more active substances such as vitamins, minerals, micronutrients, and other active substances.

In certain embodiments, the composition may contain further cosmetically active ingredients selected from plantaricin A peptide, hyaluronic acid optionally salified with sodium, potassium, calcium, a *vaccinum macrocarpon* extract obtained with the extraction technique as described above, and mixtures thereof.

According to some embodiments, the composition for oral administration is a food supplement, a nutraceutical product, a dietary product.

Food supplement means any modified food or food ingredient that can provide a benefit or protection against a skin problem or blemish.

Nutraceutical product means a product isolated or purified from edible substances.

A nutraceutical product is such when it is shown to have a physiological benefit or to provide protection against a physiological inconvenience or disorder.

Dietary or food supplement means a product that contains a biologically active substance such as a vitamin, mineral, vegetal extract, amino acid, metabolite, plant extract, concentrate, or mixture of these ingredients.

The amount administered and the frequency of administration of the composition will depend on the nature and severity of the skin blemish to be treated.

In some embodiments, the route of administration of the composition of the invention is the topical one. According to these embodiments, the composition is for topical use and is applied to the skin, in particular on face and neck.

The composition for topical application may be in solid, semi-solid or fluid form. Suitable formulations in solid form include creams, pomades, pastes, ointments.

In other embodiments, the formulation for local administration is in the fluid form, for example in the form of a lotion, suspension, or semi-fluid form, for example in the form of a serum, gel, emulsion.

Preferably the topical composition is in the fluid form of a serum, in particular for application on the face and neck. It is a form suitable for most skin types which leaves the skin dry after application. Once applied to the skin, the serum enables the *Terminalia Chebula* extract to reach the deeper layers of the epidermis, favoring the achievement of an aesthetic effect in a short treatment period, for example 5-15 days.

In some embodiments, the compositions of the invention may comprise excipients commonly used in the formulation of cosmetic or pharmaceutical preparations for local use, such as preservatives, bactericidal agents, stabilizers, emulsifiers, buffers, humectants, dyes and other excipients commonly used in the cosmetic/pharmaceutical preparation techniques.

In one embodiment, the formulation for local application is in the form of an emulsion containing the extract delivered in a suitable excipient. In some embodiments, the composition for topical application comprises an excipient of the hydroxymethylcellulose type and/or gelling agents suitable for the formulation and substances.

In applications in the cosmetic field, for example in preventing or treating skin aging, it is possible to apply a cosmetically active amount of the composition of the invention on the affected skin area one or more times a day, conveniently for a period of at least 2-3 months.

According to another aspect of the invention, a cosmetic treatment method is provided which comprises the application on the skin of an effective amount of a composition for topical use or a tannin as previously described.

The present invention will now be described with reference to the following examples which are provided for illustrative purposes only and are not to be construed as limiting the present invention.

### EXAMPLE No. 1

Composition in the form of a serum having the following qualitative-quantitative composition:

| **COMPONENT (trade name)** | **INCI** | **%** |
|---|---|---|
| Purified water | AQUA | 81.15 |
| Plantaricin | | 0.1-0.2 |
| Synastol | Terminalia Chebula Fruit Extract | 0.5-0.05 |
| Peptilium | Vaccinium macrocarpon fruit extract | 1-2 |
| CG Fibramin | Aqua, glycerin, phenoxyethanol, disodium edta, glycine soja oil, sodium oleate, hydrogenated lecithin, oligopeptide-42 | 1-2 |
| Solagum Tara | Tara gum | 0.1-0.2 |
| CARBOPOL ULTREZ 30 POLYMER | Carbomer | 0.05-0.6 |
| Aristoflex TAC | | 0.15-0.30 |
| Hyaluronic Acid Powder 1.5-2.0 MDa | Sodium hyaluronate | 0.1-0.2 |
| VEGETABLE GLYCEROL | Glycerin | 10-15 |
| Euxyl K712 | Aqua, sodium benzoate, potassium sorbate | 0.5-1 |
| Visage 2126167 | Parfum | 0.1-0.2 |

### EXAMPLE No. 2

Supplement for oral administration having the following composition:

### UNCOATED TABLET

| | |
|---|---|
| Terminalia Chebula | 5-50 mg |
| Functional substance 1 | 1-15 mg |
| Sodium croscarmellose | 10.0 mg |
| Dicalcium phosphate | 160 mg |
| Microcrystalline cellulose | 140 mg |
| Fatty acids magnesium salts | 4.0 mg |
| Silicon dioxide | 4.0 mg |

### EXAMPLE No. 3

### RETARD COATED TABLET

| | |
|---|---|
| Terminalia Chebula | 5-50 mg |
| Functional substance 1 | 1-15 mg |
| Hydroxy-propyl-methylcellulose | 18 mg |
| Dicalcium phosphate | 200 mg |
| Microcrystalline cellulose | 200 mg |
| Hydroxy-propyl-cellulose | 40.0 mg |
| Mono- and di-glycerides of fatty acids | 5.0 mg |
| Silicon dioxide | 4.0 mg |
| Film coating (Hydroxy-propyl-methylcellulose, microcrystalline cellulose Calcium carbonate, stearic acid, red iron oxide, yellow iron oxide, riboflavin) | 15 mg |

### EXAMPLE No. 4

### COATED TABLET

| | |
|---|---|
| Terminalia Chebula | 5-50 mg |
| Functional substance 1 | 1-15 mg |
| Sodium croscarmellose | 10.0 mg |
| Dicalcium phosphate | 175 mg |
| Microcrystalline cellulose | 165 mg |
| Hydroxy-propyl-cellulose | 40.0 mg |
| Mono-and di-glycerides of fatty acids | 4.8 mg |
| Silicon dioxide | 4.0 mg |
| Film coating (Hydroxy-propyl-methylcellulose, microcrystalline cellulose Calcium carbonate, stearic acid, cupric chlorophylline | 13 mg |

### EXAMPLE No. 5

### MATERIALS AND METHODS

The immortalized line of human keratinocytes NCTC 2544 (Perry V.P. et al., 1957) cultured at 37°C in a humid atmosphere with 5% CO₂ in RPMI culture medium, supplemented with 10% fetal bovine serum (FBS), 2mM glutamine, in the presence of 1% penicillin and streptomycin and 0.1% gentamicin, was used.

Chebulagic acid was tested at 0.5 mg/mL and 0.05 mg/mL and diluted in complete RPMI medium supplemented with 2.5% fetal bovine serum (FBS).

The anti-aging activity of chebulagic acid was evaluated by studying the gene expression of the Vascular endothelial growth factor A (VEGF-A) marker by relative quantitative RT-PCR (quantitative reverse transcription-polymerasechainreaction-qRT-PCR).

NCTC2544 cells were incubated for 24 and 48 hours. Cells kept in the culture medium alone (RPMI 2.5% FCS) were used as a negative control instead.

At the end of the incubation, we proceeded with RNA extraction (Chomczynski and Mackey 1995), reverse transcription into cDNA using the commercial kit "PrimeScriptTM RT Reagent Kit (perfect Real Time)" (TakaraBioInc., Japan). At the end of reverse transcription, the samples were amplified by qRT-PCR using the following TaqMan probes: Hs00900055_m1 (VEGF-A) and Hs99999905_m1 (GAPDH). GAPDH and the commercial PRIME SCRIPT RT reagent kit (TakaraBioInc., Japan) were used as the control gene (housekeeping).

The data obtained were analyzed according to the 2-ΔΔCt method and it was thus possible to calculate the relative expression values of the gene of interest, normalized with respect to the housekeeping gene and calibrated with the control sample (untreated cells)).

### RESULTS

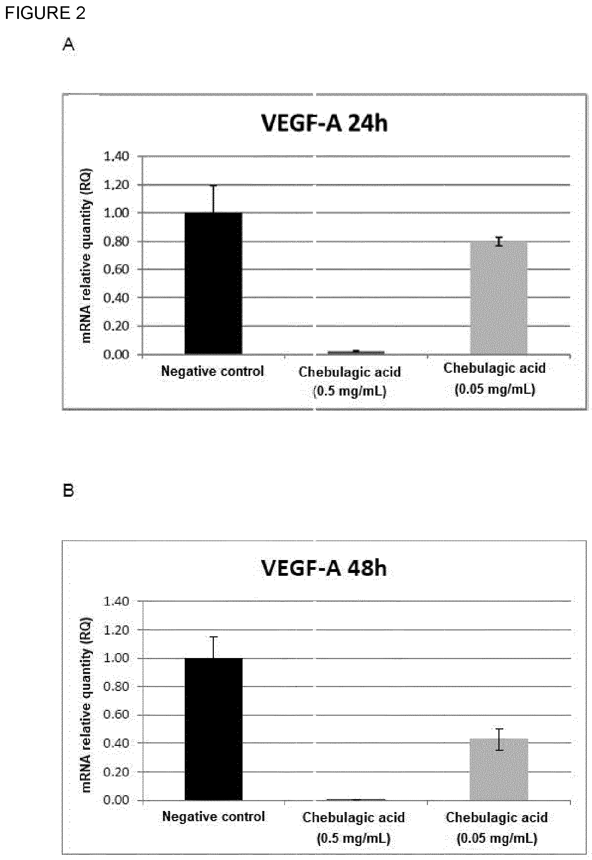

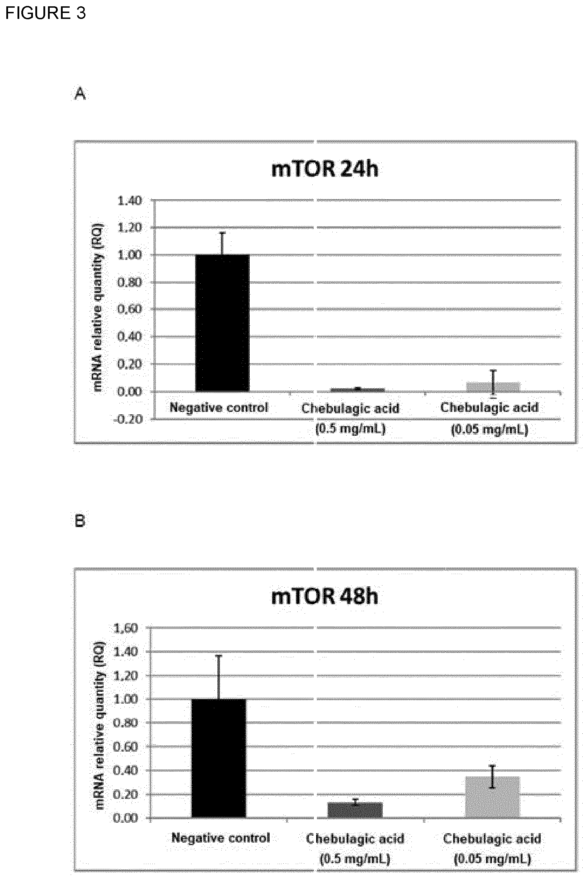

The expression levels of *VEGF-A* and *mTOR* were quantified in NCTC2544 human keratinocytes after incubation for 24 and 48h with 0.5 and 0.05 mg/mL of chebulagic acid, respectively. Chebulagic acid at 0.5 mg/mL was found to be able to significantly inhibit (P <0.05) both *VEGF-A* and *mTOR* after 24 hours of treatment (Fig. 2A and 2B). This effect persists even after 48H of treatment (Fig. 2B and 3B). Chebulagic acid significantly inhibits (p<0.005) *VEGF-A* also at the lowest concentration (0.05 mg/mL), but this effect is lower than that of the highest concentration, both after 24h and 48h of treatment (Fig. 2A and 2B). In contrast, the 0.05 mg/mL concentration of chebulagic acid exerts an effect on *mTOR* similar to that of the higher concentration after 24h of treatment (Fig. 3A). The effect is instead reduced after 48 hours of treatment, although a significance persists (P<0.01) towards the negative control (Fig. 3B).

With reference to Figure 2 on the relative quantification of *VEGF-A* at 24h A) and 48h B) of incubation with chebulagic acid in NCTC2544 human keratinocytes, the gene expression was determined by RT-PCR.

NCTC2544 human keratinocytes were treated with chebulagic acid at 0.5 and 0.05 mg/mL for 24 and 48h. Cells treated with the culture medium alone served as a negative control. The analyzes were performed after incubation at 37°C for 24 hours with 5% CO₂. The data are the means ± SD of three separate experiments performed in triplicate. Statistical differences between mean values were determined with Tukey's HSD test. The asterisk indicates a significant difference (P<0.05) with respect to the control. Statistical differences between mean values were determined by one-way ANOVA with Tukey. The symbols indicate a significant difference with respect to the control (*P<0.05; **P<0.01; ***P<0.005).

With reference to Figure 3 relating to the relative quantification of *mTOR* at 24h A) and 48h B) after incubation with chebulagic acid in NCTC2544 human keratinocytes, the gene expression was determined by RT-PCR.

NCTC2544 human keratinocytes were treated with chebulagic acid at 0.5 and 0.05 mg/mL for 24 and 48h. Cells treated with the culture medium alone served as a negative control. The analyzes were performed after incubation at 37°C for 24 hours with 5% CO₂. The data are the means ± SD of three separate experiments performed in triplicate. Statistical differences between mean values were determined with Tukey's HSD test. The asterisk indicates a significant difference (P<0.05) with respect to the control. Statistical differences between mean values were determined by one-way ANOVA with Tukey. Symbols indicate a significant difference with respect to the control (*P<0.05; **P<0.01; ***P<0.005).

### DISCUSSIONS AND CONCLUSIONS

Chebulagic acid, in particular from *Terminalia Chebula,* at concentrations of 0.5 and 0.05 mg/mL is able to significantly inhibit the expression of *VEGF-A* and *mTOR* in the NCTC2544 human keratinocyte model after 24 and 48h of treatment. This effect translates into an effective anti-aging action.

### BIBLIOGRAPHY

1. .... A Keren, L Yndriago, A Izeta, D Gerovska, M Araúzo-Bravo, R Paus and A Gilhar et al. 1429 VEGF mediates rejuvenation of aged human skin xenografts in young mice. Journal of investigative dermatology, Vol.138(5), pp.S242-S242; 2018-05.
2..... Lu, K., Basu, S. The natural compound chebulagic acid inhibits vascular endothelial growth factor A mediated regulation of endothelial cell functions. Sci Rep 5, 9642 (2015). https://doi.org/10.1038/srep09642
3..... Papadopoli D, Boulay K, Kazak L, et al. mTOR as a central regulator of lifespan and aging. F1000Res. 2019;8:F1000 Faculty Rev-998. Published 2019 Jul 2. doi:10.12688/f1000research.17196.1)
4..... Neufahrt A, Förster FJ, Heine H, Schaeg G, Leonhardi G. Long-term tissue culture of epithelial-like cells from human skin (NCTC strain 2544). I. Measurement of viscosity. Arch Dermatol Res. 1976 Oct 27;256(3):255-60. doi: 10.1007/BF00572490. PMID: 990102
5..... Chomczynski P, Mackey K. Modification of the TRI reagent procedure for isolation of RNA from polysaccharide- and proteoglycan-rich sources. Biotechniques;1995;19:942-5.
6..... Vigetti D, Viola M, Karousou E, Rizzi M, Moretto P, Genasetti A, et al. Hyaluronan-CD44-ERK1/2 regulate human aortic smooth muscle cell motility during aging. J Biol Chem 2008;283:4448-58.
Mosmann T, 1983. Rapid Colorimetric Assay for Cellular Growth and Survival: Application to Proliferation and Cytotoxicity Assays. J Immunol Methods 65(1-2), 55-63.
Coda R, Rizzello CG, Pinto D, Gobbetti M, 2012. Selected Lactic Acid Bacteria Synthesize Antioxidant Peptides during Sourdough Fermentation of Cereal Flours. Appl Environ Microbiol 78(4), 1087-1096.
Chomczynski P, Mackey K. Modification of the TRI reagent procedure for isolation of RNA from polysaccharide- and proteoglycan-rich sources. Biotechniques 1995;19:942-5.
or signs of skin aging.

## Claims

1. A non-therapeutic cosmetic use of a composition comprising a tannin and a physiologically acceptable carrier in preventing or reducing aesthetic skin damage or signs of skin aging, **characterized in that** the tannin comprises chebulagic acid and that said composition is a topical composition and comprises plantaricin, *Vaccinum macrocarpon* extract and hyaluronic acid or a physiologically acceptable salt thereof.

2. The use according to claim 1, wherein the chebulagic acid is contained in a *Terminalia Chebula* plant extract or is obtainable by extraction from *Terminalia Chebula* plant.

3. The use according to claim 1 or 2, wherein the tannin comprises chebulinic acid.

4. The use according to claim 2 or 3, wherein the *Terminalia Chebula* extract is obtained by extraction from fruits or leaves of *Terminalia Chebula* plant with a physiologically acceptable solvent.

5. The use according to any one of claims 2-4, wherein the *Terminalia Chebula* extract is obtainable by extraction with a physiologically acceptable polar protic solvent.

6. The use according to claim 5, wherein the polar protic solvent is selected from the group comprising water, ethyl alcohol and mixtures thereof, and is preferably water.

7. The use according to any one of claims 1-6, **characterized in that** the composition is in a fluid form, preferably in the form of a cosmetic serum.

8. The use according to any one of claims 1-7, wherein the signs of skin aging are wrinkles, skin roughness, skin thickening, dehydration, creasing, sagging skin, photoaging.

9. A non-therapeutic cosmetic use according to claim 1 of chebulagic acid, or a *Terminalia Chebula* plant extract titrated in chebulagic acid, to prevent or reduce aesthetic skin damage or signs of skin aging.

## Patentansprüche

1. Nicht-therapeutische, kosmetische Verwendung einer Zusammensetzung, die ein Tannin und einen physiologisch verträglichen Träger umfasst, zur Verhinderung oder Verringerung ästhetischer Hautschäden oder Zeichen der Hautalterung, **dadurch gekennzeichnet, dass** das Tannin Chebulagsäure umfasst und dass die Zusammensetzung eine topische Zusammensetzung ist und Plantaricin, *Vaccinum-macrocarpon*-Extrakt und Hyaluronsäure oder ein physiologisch verträgliches Salz davon umfasst.

2. Verwendung nach Anspruch 1, wobei die Chebulagsäure in einem Terminalia-Chebula-Pflanzenextrakt enthalten ist oder durch Extraktion aus der *Terminalia-Chebula*-Pflanze gewonnen werden kann.

3. Verwendung nach Anspruch 1 oder 2, wobei das Tannin Chebulinsäure umfasst.

4. Verwendung nach Anspruch 2 oder 3, wobei der *Terminalia-Chebula*-Extrakt durch Extraktion aus Früchten oder Blättern der *Terminalia-Chebula*-Pflanze mit einem physiologisch verträglichen Lösungsmittel gewonnen wird.

5. Verwendung nach einem der Ansprüche 2-4, wobei der *Terminalia-Chebula-*Extrakt durch Extraktion mit einem physiologisch verträglichen polaren protischen Lösungsmittel gewonnen werden kann.

6. Verwendung nach Anspruch 5, wobei das polare protische Lösungsmittel aus der Gruppe ausgewählt ist, die Wasser, Ethylalkohol und Mischungen davon umfasst, und vorzugsweise Wasser ist.

7. Verwendung nach einem der Ansprüche 1-6, **dadurch gekennzeichnet, dass** die Zusammensetzung in Fluidform vorliegt, vorzugsweise in der Form eines kosmetischen Serums.

8. Verwendung nach einem der Ansprüche 1-7, wobei die Zeichen der Hautalterung Falten, Hautrauhigkeit, Hautverdickung, Dehydrierung, Fältchenbildung, schlaffe Haut und Lichtalterung sind.

9. Nicht-therapeutische, kosmetische Verwendung nach Anspruch 1 von Chebulagsäure oder eines *Terminalia-Chebula*-Pflanzenextrakts, der in Chebulagsäure titriert wird, um ästhetische Hautschäden oder Zeichen der Hautalterung zu verhindern oder zu verringern.

## Revendications

1. Utilisation cosmétique non thérapeutique d'une composition comprenant un tanin et un support physiologiquement acceptable pour prévenir ou réduire les dommages esthétiques de la peau ou les signes de vieillissement cutané, **caractérisée en ce que** le tanin comprend de l'acide chébulagique et **en ce que** ladite composition est une composition topique et comprend de la plantaricine, de l'extrait de *Vaccinum macrocarpon* et de l'acide hyaluronique ou un sel physiologiquement acceptable de celui-ci.

2. Utilisation selon la revendication 1, dans laquelle l'acide chébulagique est contenu dans un extrait de plante *Terminalia Chebula* ou est obtenable par extraction à partir de la plante *Terminalia Chebula.*

3. Utilisation selon la revendication 1 ou 2, dans laquelle le tanin comprend de l'acide chébulinique.

4. Utilisation selon la revendication 2 ou 3, dans laquelle l'extrait de *Terminalia Chebula* est obtenu par extraction à partir de fruits ou de feuilles de plante *Terminalia Chebula* avec un solvant physiologiquement acceptable.

5. Utilisation selon l'une quelconque des revendications 2 à 4, dans laquelle l'extrait de *Terminalia Chebula* peut être obtenu par extraction avec un solvant protique polaire physiologiquement acceptable.

6. Utilisation selon la revendication 5, dans laquelle le solvant protique polaire est choisi dans le groupe comprenant l'eau, l'alcool éthylique et leurs mélanges, et il est de préférence eau.

7. Utilisation selon l'une quelconque des revendications 1 à 6, **caractérisée en ce que** la composition est sous forme fluide, de préférence sous forme d'un sérum cosmétique.

8. Utilisation selon l'une quelconque des revendications 1 à 7, dans laquelle les signes du vieillissement cutané sont les rides, la rugosité de la peau, l'épaississement de la peau, la déshydratation, le plissement, la peau relâchée, le photovieillissement.

9. Utilisation cosmétique non thérapeutique selon la revendication 1 d'acide chébulagique, ou d'un extrait de plante *Terminalia Chebula* titré en acide chébulagique, pour prévenir ou réduire les dommages esthétiques cutanés ou les signes de vieillissement cutané.
